# EUROPEAN PATENT SPECIFICATION

(11) **EP 1 606 236 B1**
(45) Date of publication and mention of the grant of the patent: **04.09.2013**
(21) Application number: 04716882.8
(22) Date of filing: 03.03.2004
(51) Int. Cl.: C07C 7/09, C07C 9/06, C10L 3/10, F25J 3/02

(54) **RESIDUE RECYCLE-HIGH ETHANE RECOVERY PROCESS**
ETHAN WIEDERGEWINNUNGSPROZESS AUS RÜCKSTÄNDEN
PROCEDE DE RECUPERATION D'ETHANE A RECYCLAGE ELEVE DE RESIDU

(30) Priority: 07.03.2003 US 453072 P
(43) Date of publication of application: 21.12.2005
(73) Proprietor: Lummus Technology Inc., Houston TX 77042 (US)
(72) Inventor: PATEL, Sanjiv, N., Sugar Land, TX 77478 (US); FOGLIETTA, Jorge, H., Missouri City, TX 77459 (US)
(74) Representative: Shaw, Matthew Nigel
(86) International application number: PCT/US2004/006418
(87) International publication number: WO 2004/080936

(56) References cited:
- WO-A-99/23428
- US-A- 4 889 545

## Description

### Related Applications

This patent application claims priority to U.S. Provisional Patent Application Serial No. 60/453,072 filed on March 7, 2003.

### BACKGROUND OF THE INVENTION

### Technical Field of the Invention

The present invention relates to the recovery of ethane compounds from hydrocarbon gas streams. More particularly, the present invention relates to the recovery of ethane compounds from hydrocarbon inlet gas streams using multiple reflux streams.

### Description of Prior Art

Many prior art processes exist WO 99/23428 for the recovery of ethane compounds from hydrocarbon inlet gas streams. An example ethane recovery process can be found in U.S. Patent No. 5,890,377 issued to Foglietta. Residue recycle processes are capable of obtaining high ethane recoveries (in excess of 95 %), while recovering essentially 100 % of C3+. Such processes, though impressive in achieving high recoveries consume a lot of energy in terms of compression. In order to reduce energy consumption while still maintaining high recoveries, an additional source of reflux is required. The requirements for this reflux stream are that it should be lean in desirable components (C2+) and be available at a high pressure. Prior art schemes have identified some alternate sources of reflux. The process disclosed here has a unique way of obtaining such a reflux stream. This reflux stream is used as intermediate reflux thereby reducing flow of the main reflux stream and hence energy consumption. In this process, an inlet gas is cooled by heat exchange with other streams in the process, without first splitting the inlet gas stream. As the inlet gas stream is cooled, liquid can be condensed and separated to form a first liquid stream and a first vapor stream. The first vapor stream is expanded in a turboexpander to further cool the stream. The cooled stream is then introduced to a demethanizer column at an intermediate feed position. The first liquid portion from the separator is expanded and directed to the demethanizer at a relatively lower feed position. The overhead stream from the demethanizer is heated, and compressed to a higher pressure and then divided into a volatile gas residue fraction and a compressed recycle stream. The compressed recycle stream is cooled sufficiently to substantially condense it by contacting it with the side reboilers as a part of the demethanizer column. The compressed recycle stream is further cooled and expanded to a lower pressure and supplied to the demethanizer column at a top feed position to reflux the column. The Foglietta process described above achieves a relatively high recovery efficiency of 95% and greater for ethane and heavier compounds.

A need exists for an ethane recovery process that is capable of achieving a recovery efficiency of at least 95%, but with lower energy consumption compared to prior art processes. A need also exists for a process that can take advantage of temperature profiles within a process to reduce the amount of external energy requirements that are needed to achieve high recovery efficiencies.

### SUMMARY OF INVENTION

In order to meet one or more of these goals, the present invention advantageously includes a process according to claim 1 and apparatus according to claim 10 for ethane recovery with a decrease in compression requirements for residue gas while maintaining a high recovery yield of ethane ("C₂+") compounds from a hydrocarbon inlet gas stream. The inlet gas stream is split into two streams. The first feed stream is cooled by heat exchange contact in a front-end exchanger and the second feed stream is cooled by heat exchange contact in the one or more reboilers of a fractionation tower. The fractionation tower can be a demethanizer tower or any suitable device capable of recovering ethane and heavier components at a bottom of the tower from a hydrocarbon inlet gas. The two feed streams are then directed into a cold absorber. The cold absorber preferably contains at least two packed beds, or other mass transfer zones, within the cold absorber. Mass transfer zones can include any type of device that is capable of transferring molecules from a liquid flowing down the vessel containing the mass transfer zone to a gas rising through the vessel and from the gas rising through the vessel to the liquid flowing down the vessel. Other types of mass transfer zones will be known to those skilled in the art and are to be considered within the scope of the present invention. Two separate vessels with packed beds can also be used as the cold absorber instead of having a single vessel with two packed beds. The colder stream of the two streams is introduced at the top of the cold absorber, preferably above a top or first mass transfer zone, while the warmer stream is sent to the bottom of the cold absorber, preferably below a bottom or second mass transfer zone.

The cold absorber produces an absorber overhead stream, an absorber bottoms stream, and an absorber side draw stream. The absorber bottoms stream is directed to the fractionation tower as a third fractionation tower feed stream. The absorber overhead stream is sent to an expander and then to the fractionation tower as a second fractionation tower feed stream. A residue recycle stream is also sent to the fractionation tower, preferably at a top location on the fractionation tower. The residue recycle stream is taken as a split of a residue gas stream. The residue gas stream is formed by warming and then compressing a fractionation tower overhead stream. The residue recycle stream is cooled and substantially condensed prior to being sent to the fractionation tower.

The absorber side draw stream is preferably removed from between the two mass transfer zones. The absorber side draw stream is then condensed and sent to the fractionation tower. The absorber side draw stream can be sent to the fractionation tower below the residue recycle stream as an intermediate feed stream. Alternatively, the tower side draw stream can be added to the residue recycle stream to form the first fractionation tower feed stream. The alternate embodiment is particularly effective when a lean hydrocarbon feed stream is used.

The fractionation tower also produces one or more reboiler streams and a fractionation tower bottoms stream. The reboiler streams are warmed in a reboiler and redirected back to the fractionation tower to supply heat to the fractionation tower and recover refrigeration effects from the fractionation tower. The fractionation tower bottoms stream contains the major portion of the recovered C₂+ compounds. The recovery of the C₂+ compounds is comparable to other C₂+ recovery processes, but the compression requirements are much lower.

### BRIEF DESCRIPTION OF THE DRAWINGS

So that the manner in which the features, advantages and objects of the invention, as well as others which will become apparent, may be understood in more detail, more particular description of the invention briefly summarized above may be had by reference to the embodiment thereof which is illustrated in the appended drawings, which form a part of this specification. It is to be noted, however, that the drawings illustrate only a preferred embodiment of the invention and is therefore not to be considered limiting of the invention's scope as it may admit to other equally effective embodiments.

FIG. 1 is a simplified flow diagram of a typical C₂+ compound recovery process, in accordance with a prior art process;

FIG. 2 is a simplified flow diagram of a C₂+ compound recovery process that incorporates the improvements of the present invention and is configured for reduced compression requirements while maintaining a high recovery of C₂+ from a hydrocarbon gas stream through the use of a side stream taken from a cold absorber and sending the stream to the fractionation tower according to an embodiment of the present invention;

FIG. 3 is a simplified flow diagram of a C₂+ compound recovery process that incorporates the improvements of the present invention and is configured for reduced compression requirements while maintaining a high recovery of C₂+ compounds through the use of an alternate feed configuration for the cold absorber side stream to the fractionation tower according to an embodiment of the present invention; and

FIG. 4 is a simplified diagram illustrating an optional feed configuration for the hydrocarbon feed streams sent to a cold absorber according to an embodiment of the present invention.

### DETAILED DESCRIPTION OF THE DRAWINGS

For simplification of the drawings, figure numbers are the same in the figures for various streams and equipment when the functions are the same, with respect to the streams or equipment, in each of the figures. Like numbers refer to like elements throughout, and prime, double prime, and triple prime notation, where used, generally indicate similar elements in alternative embodiments.

As used herein, the term "inlet gas" means a hydrocarbon gas, such gas is typically received from a high-pressure gas line and is substantially comprised of methane, with the balance being C₂ compounds, C₃ compounds and heavier compounds as well as carbon dioxide, nitrogen and other trace gases. The term "C₂ compounds" means all organic compounds having two carbon atoms, including aliphatic species such as alkanes, olefins, and alkynes, particularly, ethane, ethylene, acetylene, and the like. The term "C₂+ compounds" means all C₂ compounds and heavier compounds.

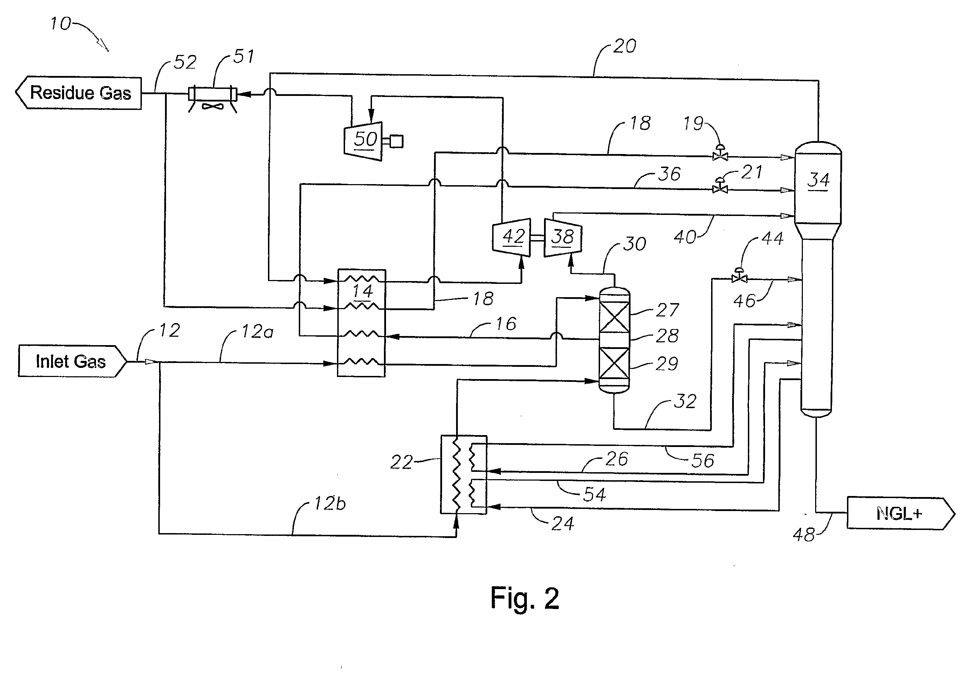

FIG. 2 illustrates one embodiment of the improved C₂+ compound recovery scheme 10. The present invention advantageously provides a process for separating an inlet gas stream 12 containing methane, C2 components, C3 components and heavier hydrocarbons into a volatile gas fraction containing substantially all the methane and a less volatile hydrocarbon fraction containing a large portion of the C2+ components. Inlet gas stream 12 is split into a first feed stream 12a and a second feed stream 12b. A preferable split of the inlet gas stream 12 is about 70% as first feed stream 12a and the remainder going to second feed stream 12b. However, the split between first and second feed streams 12a and 12b can vary depending upon the duty available from a fractionation tower 34. Fractionation tower 34 can be a demethanizer tower or any other suitable device that can recover ethane and heavier components from the inlet gas stream. Other suitable devices will be known to those skilled in the art and are to be considered within the scope of the present invention.

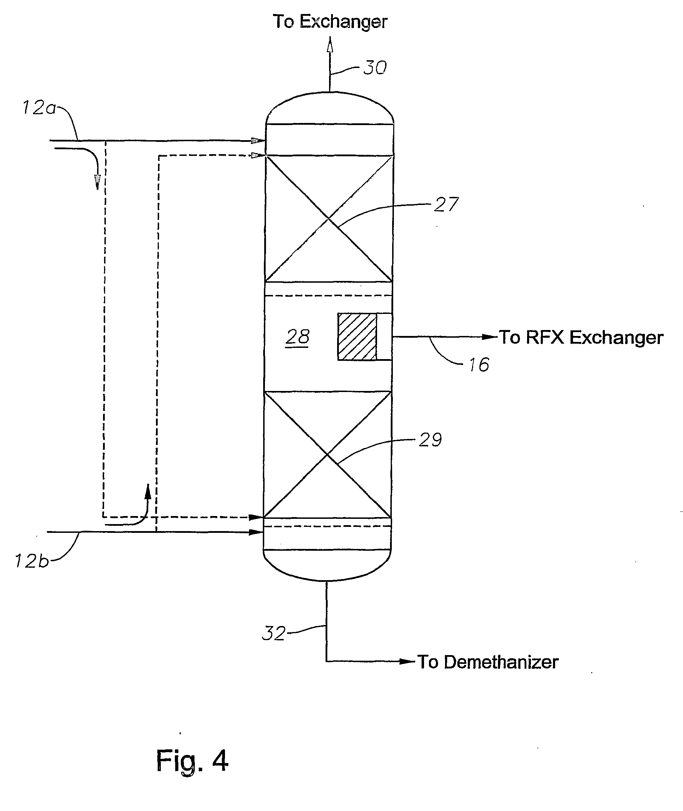

First feed stream 12a is cooled in front end exchanger 14 preferably by heat exchange contact with at least one of an absorber side draw stream 16, a residue recycle stream 18, a fractionation tower overhead stream 20, and combinations thereof to at least partially condense first feed stream 12a. Second feed stream 12b is cooled in a fractionation tower reboiler 22 preferably by heat exchange contact with a first reboiler stream 24 and preferably a second reboiler stream 26. First feed stream 12a and second feed stream 12b can be cooled by other heat exchange contact means, as understood by those of ordinary skill in the art and are to be considered within the scope of the present invention. In all embodiments of this invention, front-end exchanger 14 and fractionation tower reboiler 22 can be a single multi-path exchanger, a plurality of individual heat exchangers, or combinations and variations thereof. First and second feed streams 12a, 12b are sent to a cold absorber 28. Cold absorber 28 preferably includes at least two packed beds, or mass transfer zones or units, 27 and 29. Two separate vessels with packed beds can also be used instead of a single vessel with both packed beds contained within. Mass transfer zones can include any type of device that is capable of transferring molecules from a liquid flowing down the vessel containing the mass transfer zone to a gas rising through the vessel and from the gas rising through the vessel to the liquid flowing down the vessel. Other types of mass transfer zones will be known to those skilled in the art and are to be considered within the scope of the present invention. As shown in FIG. 4, the colder of two feed streams 12a, 12b is sent to the top of cold absorber 28, above or before first packed bed 27, with the warmer of the two feed streams being sent to the bottom of cold absorber 28, below or after second packed bed 29. FIG. 4 shows a bypass option to allow for directing of first and second feed streams 12a and 12b to cold absorber top or bottom depending upon temperature.

Cold absorber 28, shown in FIG. 2, produces an absorber overhead stream 30, an absorber bottoms stream 32, and absorber side draw stream 16. Cold absorber 28 preferably contains at least two packed beds 27, 29, or mass transfer zones or units, within cold absorber 28. As an improvement to prior art processes, a cold absorber is used instead of a cold separator. Absorber side draw stream 16 is taken from the packed bed cold absorber 28 preferably between the two packed beds 27, 29. Tower side draw stream 16 is then substantially condensed in front end exchanger 14 and sent to fractionation tower 34 as intermediate tower feed stream 36. Because of the substantial condensation, in some embodiments, intermediate tower feed stream 36 can be substantially liquid. Intermediate tower feed stream 36 is preferably fed to fractionation tower 34 at a location below residue recycle stream 18.

Prior art processes attempted to control the temperatures of feed streams 12a and 12b to essentially be the same to minimize energy losses due to the different temperature mix. With the present invention, there can be a temperature difference between the streams of up to about 15°F without affecting the efficiency of the process and simultaneously decreasing the compression requirements of residue gas stream 52 of the process. The colder of the two streams is sent to the top of the cold absorber 28 with the warmer of the two streams being sent to the bottom of the cold absorber 28. The mass transfer zones 27, 29 within the cold absorber 28 work with the differences in temperatures to equalize the temperatures of the two streams. The temperature of the side draw stream 16 will be in between the temperatures of top and bottom streams and the composition will be leaner than both feed streams.

To decrease the compression requirements of residue gas stream 52, intermediate tower feed stream 36 provides a secondary reflux source to supply to fractionation tower 34. The secondary reflux source allows for a reduction in the amount of material refluxed back to fractionation tower 34 in residue recycle stream 18. The less material required in residue recycle stream 18', the less material that has to be compressed in residue gas stream 52, which decreases the compression requirements for this stream. The recovery of the process remains the same as in prior art processes.

Absorber overhead stream 30 is expanded in expander 38 and sent or supplied to fractionation tower 34, preferably to a position below intermediate tower feed stream 36, as second fractionation tower feed stream 40. During the expansion, the temperature of absorber overhead stream 30 is lowered and work is produced. This work is later recovered in a booster compressor 42 driven by the expander 38 to partially boost pressure of fractionation tower overhead stream 20.

Absorber bottoms stream 32 can be expanded through expansion valve 44 or the like and is sent to fractionation tower 34 as a third fractionation tower feed stream 46. In this embodiment, fractionation tower 34 is also supplied second fractionation tower feed stream 40, residue recycle stream 18, and intermediate tower feed stream 36, thereby producing fractionation tower overhead stream 20, a fractionation tower bottoms stream 54, and reboiler bottoms streams 24 and 26.

In fractionation tower 34, desired components (C2+) in the rising are at least partially condensed by intimate contact with falling, thereby producing the fractionation tower overhead stream 20 that contains substantially all of the methane and lighter or non-condensable components. The condensed liquids descend down fractionation tower 34 and are removed as fractionation tower bottoms stream 48, which contains a major portion of the C₂ components and heavier components, i.e., substantially all of the C2+ components. In other words, fractionation tower 34 separates the streams that are fed to it into fractionation tower overhead stream 20 and fractionation tower bottoms stream 48.

Reboiler streams 24, 26, are preferably removed from fractionation tower 34 in the lower half of the vessel. Reboiler streams 24, 26 are warmed in reboiler 22 and returned to fractionation tower 34 as reboiler reflux streams 54 and 56. Reboiler reflux streams 54, 56 supply heat to fractionation tower 34 and recover refrigeration from fractionation tower 34.

Fractionation tower overhead stream 20 is warmed in front end exchanger 14 and compressed in booster compressor 42 and residue compressor 50 to pipeline specifications or higher to form residue gas stream 52. Residue gas stream 52 is a pipeline sales gas that contains substantially all of the methane in the inlet gas, and a minor portion of C₂ compounds and heavier compounds. At least a portion of residue gas stream 52 is removed and cooled in front end exchanger 14 and supplied to fractionation tower 34 as residue recycle stream 18.

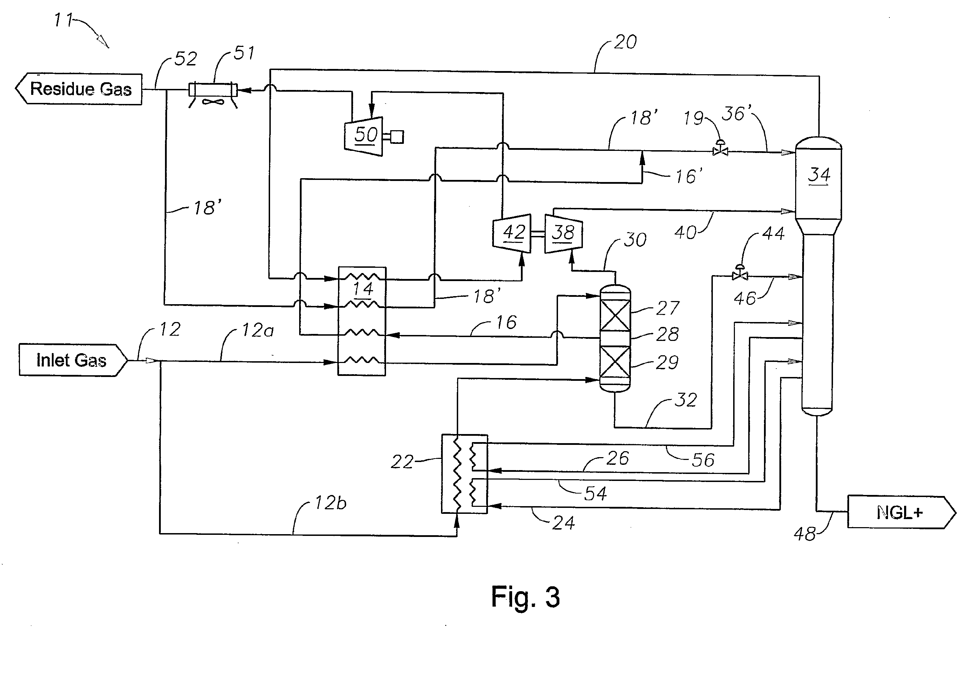

FIG. 3 depicts an alternate embodiment of the present invention. C₂+ recovery process 11, includes adding absorber side draw stream 16' to residue recycle stream 18' to form first fractionation tower feed stream 36'. First fractionation tower feed stream 36' is preferably introduced to fractionation tower 34 in a top section of fractionation tower 34. The embodiment of the present invention shown in FIG. 3 is preferable when the inlet gas stream 12 is lean. When inlet gas stream 12 is lean, to maintain recovery of the desired products, more reflux is required to be sent to the top of fractionation tower 34. More reflux to fractionation tower 34 generally requires more compression of the residue gas stream to produce more residue recycle stream 18'. If absorber side draw stream 16' is added to residue recycle stream 18', less residue recycle stream 18' and less residue gas stream 52 is needed, which lowers the compression requirements of the residue gas stream 52.

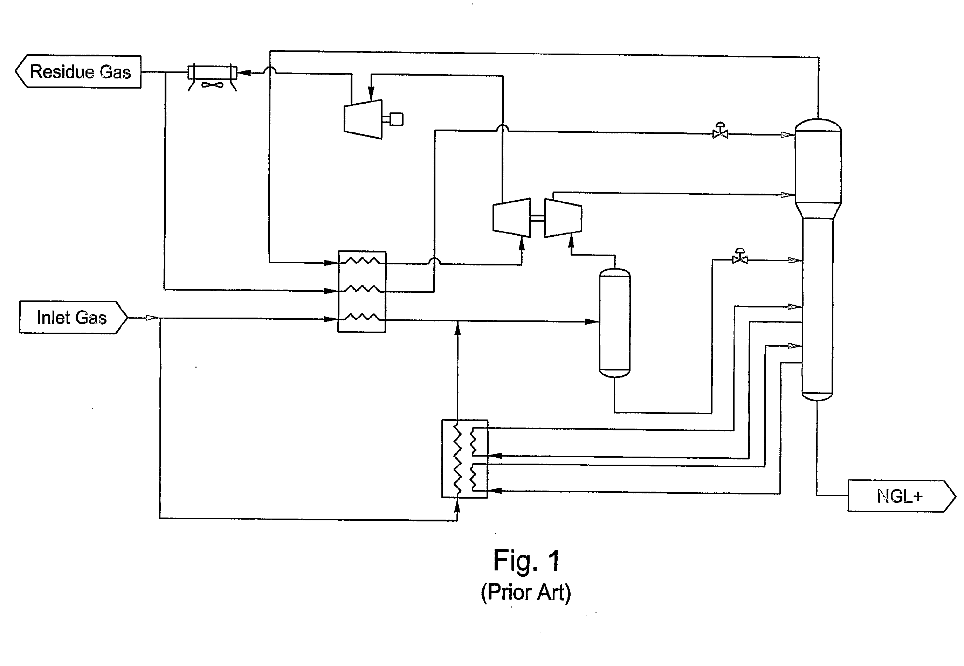

Simulations have been carried out to compare schemes shown in Figures 1 and 2. The schemes shown in the figures illustrate a single exchanger to heat and cool streams. However, the simulation model includes several heat exchangers for stream cooling and heating, which is more representative of an actual plant. Feed conditions and composition are listed below in Table 1.

| Table 1 | |
|---|---|
| Component | Mol % |
| Nitrogen | 0.15 |
| CO2 | 0.34 |
| Methane | 87.718 |
| Ethane | 6.821 |
| | |
| Propane | 2.733 |
| i-Butane | 0.792 |
| n-Butane | 0.641 |
| i-Pentane | 0.201 |
| n-Pentane | 0.252 |
| n-Hexane | 0.353 |
| Lbmol/hr | 100,000 |
| Temperature, °F | 90 |
| Pressure, psia | 800 |

| Table 2 | | |
|---|---|---|
| Item | FIG. 1 | FIG. 2 |
| C2 Recovery, % | 95 | 95.04 |
| C3+ Recovery, % | 100 | 99.96 |
| | | |
| Total Compression, hp | 56018 | 47684 |
| Total Duty, btu/h-F | 3.256E+07 | 2.944E+07 |

As can be seen in Table 2, which compares the results from simulations for FIGS. 1 and 2, the new process requires less overall compression, and lower total exchanger duty. This lower duty is mainly due to a significant decrease in residue recycle flow. The decrease in compression has two advantages. The first is lower capital cost and the second is lower operating cost. At a rate of 3.5 $/MMBtu for fuel gas, the fuel gas savings is about $2MM per year. Although the new process requires a slightly larger cold separator, or a cold absorber, the cost of this vessel is much less than the savings in capital achieved with lower compression and required heat exchanger area. Overall, the process disclosed has lower capital and operating costs than prior art referenced.

The selection of a processing scheme between Figures 2 and 3 will depend on the feed composition. The compression requirement reduction will be similar in both embodiments of the present invention. Absorber side draw stream 16 provides a secondary source for reflux to fractionation tower 34, thereby reducing the amount of residue gas 52 that is being returned to fractionation tower 34. Since less residue recycle gas 18 is sent to fractionation tower 34, less residue gas stream 52 is required to be compressed, which reduces the compression requirements for the process.

In most prior C₂+ recovery processes, process designers attempt to make the temperatures of the split inlet feed streams the same in order to minimize energy losses due to the different temperatures of the inlet feed stream when mixed together. With the use of the packed beds, only a minimum difference in temperature is needed to achieve the same C₂+ recovery. This difference makes the process easy to operate, which is another advantage of the present invention. The different temperatures of the two streams are used to produce the two feeds to the cold absorber, each with a different temperature. An absorber side draw stream 16, which has a temperature between the temperatures of the first and second feed inlet gas streams, is sent to fractionation tower 34.

In addition to the process embodiments advantageously provide, the present invention also includes an apparatus embodiment for performing the processes described herein. As an embodiment of the present invention, an apparatus for separating an inlet gas stream containing methane, C2 components, C3 components and heavier hydrocarbons into a volatile gas fraction containing substantially all the methane and a less volatile hydrocarbon fraction containing a large portion of the C2+ components is advantageously provided. The apparatus preferably includes a first cooler 14, a packed bed cold absorber 28, a first expander 38, a fractionation tower 34, a first heater I4, a first compressor 42, a second cooler 14, and a third cooler 14.

First cooler, or front end cooler, 14 is preferably used for cooling a first feed stream 12a and a second feed stream 12b. Packed bed cold absorber 28 is preferably used for receiving the first feed stream 12a and the second feed stream 12b where first feed stream 12a has a temperature colder than second feed stream 12b.

Absorber 28 preferably includes at least a first and a second packed bed 27, 29 and produces an absorber overhead stream 30, an absorber bottoms stream 32, and an absorber side draw stream 16. As indicated previously, absorber side draw stream 16 is preferably removed from absorber 28 between the first and the second packed beds 27,29.

First expander 38 preferably expands absorber overhead stream 30. During the expansion, the temperature of absorber overhead stream 30 is lowered and work is produced. This work is later recovered in a booster compressor 42 driven by the expander 38 to partially boost pressure of fractionation tower overhead stream 20.

Fractionation tower 34 separates a first fractionation tower feed stream 36, the absorber overhead stream as a second fractionation tower feed stream 40, the absorber bottoms stream as a third fractionation tower feed stream 46, and a fractionation tower reflux stream 18 to produce a fractionation tower overhead stream 20 that contains substantially all the methane and lighter components and a fractionation tower bottoms stream 48 that contains substantially all the C2+ components.

First heater 14 preferably warms the fractionation tower overhead stream. First compressor 42 compresses fractionation tower overhead stream 20 to produce a residue gas stream 52. Second cooler 14 preferably cools at least a portion of the residue gas stream 18. Third cooler 14 preferably cools and at least partially condenses absorber side draw stream 16 to form, or produce, first fractionation tower feed stream 36.

The apparatus embodiment of the present invention can also advantageously include a fourth cooler, or fractionation tower reboiler, 22 for cooling and at least partially condensing at least a portion of the inlet gas stream 12b. Fourth cooler 22 can also provide reboiler duty to fractionation tower 34 by providing heat exchange contact between at least a portion of the inlet gas stream 12b and first and second reboiler streams 24, 26.

In all embodiments of the present invention, first cooler, the second cooler, the third cooler, and the first heater can be a single heat exchanger that provides heat exchange contact between first feed stream 12a, absorber side draw stream 16, residue recycle stream 18, fractionation tower overhead stream 20, and combinations thereof.

The apparatus embodiments of the present invention can also include a second expander 44 for expanding at least a portion of the absorber bottoms stream prior to being sent to the fractionation tower. The apparatus embodiments can also include a third expander 19 for expanding at least a portion of the residue recycle stream prior to being sent to the fractionation tower. A fourth expander 21 can also be provided for expanding absorber side draw stream 16.

While the invention has been shown or described in only some of its forms, it should be apparent to those skilled in the art that it is not so limited, but is susceptible to various changes without departing from the scope of the invention.

For example, the expanding steps, preferably by isentropic expansion, may be effectuated with a turbo-expander, Joule-Thompson expansion valves, a liquid expander, a gas or vapor expander or the like. As another example, the packed beds within the packed bed tower can be filled with various types of packing, such as Racshig rings, Lessing rings, Berl saddles, or the like. The packed beds could also be filled with various types of trays, such as bubble cap trays, sieve trays, valve trays, and the like.

## Claims

1. A process for separating an inlet gas stream containing methane, C2 components, C3 components and heavier hydrocarbons into a volatile gas fraction containing substantially all the methane and a less volatile hydrocarbon fraction containing at least 95% of the C2+ components, the process comprising the steps of:
(a) splitting an inlet gas stream into a first feed stream and a second feed stream and cooling the first and the second feed streams;
(b) supplying a top of a packed bed cold absorber with the first feed stream and a bottom of the packed bed cold absorber with the second feed stream where the first feed stream has a temperature colder than the second feed stream, the packed bed cold absorber comprising at least a first and a second packed bed and producing an absorber overhead stream, an absorber bottoms stream, and an absorber side draw stream; .
(c) removing the absorber side draw stream from the packed bed cold absorber;
(d) cooling and at least partially condensing the absorber side draw stream to form a first fractionation tower feed stream:
(e) expanding and then supplying a fractionation tower with the absorber overhead stream as a second fractionation tower feed stream;
(f) supplying the fractionation tower with the absorber bottoms stream as a third fractionation tower feed stream;
(g) separating a first fractionation tower feed stream, the second fractionation tower feed stream, the third fractionation tower, and the fractionation tower reflux stream to produce a fractionation tower overhead stream that contains substantially all the methane and lighter components and a fractionation tower bottoms stream that contains at least 95% of the C2+ components;
(h) wanning and compressing the fractionation tower overhead stream to produce a residue gas stream;
(i) removing at least a portion of the residue gas stream as a fractionation tower reflux stream;
(j) cooling and supplying the fractionation tower reflux stream to the fractionation tower as a residue recycle stream; and
(k) supplying the fractionation tower with the first fractionation tower feed stream thereby reducing an amount of the residue gas being compressed and an amount of the residue recycle gas being sent to the fractionation tower.

2. The process of claim 1, wherein the step of removing the absorber side draw stream from the packed bed cold absorber includes removing the absorber side draw stream between the first and the second packed beds.

3. The process of claim 1 or claim 2, wherein the step of splitting the inlet gas stream includes splitting the inlet gas stream so that the first feed stream contains about 70% of the inlet gas stream and the second feed stream contains about 30% of the inlet gas stream.

4. The process of any one of claims 1 to 3, wherein the step of cooling the first and second feed streams includes the steps of:
(a) cooling the first feed stream by heat exchange contact with a stream selected from the group consisting of the absorber side draw stream, the residue recycle stream, the fractionation tower overhead stream, and combinations thereof; and
(b) cooling the second feed stream by heat exchange contact with a stream selected from the group consisting of a first reboiler bottoms stream, a second reboiler bottoms stream, and combinations thereof.

5. The process of any one of claims 1 to 4, wherein the step of cooling and at least partially condensing the absorber side draw stream includes cooling the absorber side draw stream so that substantially all of the absorber side draw stream is in the liquid phase.

6. The process of any one of claims 1 to 5, further including the step of expanding the absorber bottoms stream prior to supplying it to the fractionation tower.

7. The process of any one of claims 1 to 6, further including the step of expanding the absorber side draw stream prior to supplying it to the fractionation tower.

8. The process of any one of claims 1 to 7, further including the step of expanding the residue recycle stream prior to supplying it to the fractionation tower.]

9. A process according to any one of claims 1 to 8, further including the step of adding the absorber side draw stream to the residue recycle stream to form the first fractionation tower feed stream.

10. An apparatus for separating an inlet gas stream containing methane, C2 components, C3 components and heavier hydrocarbons into a volatile gas fraction containing substantially all the methane and a less volatile hydrocarbon fraction containing at least 95% of the C2+ components, the apparatus comprising:
(a) a first cooler for cooling a first feed stream and a second feed stream;
(b) a packed bed cold absorber for receiving the first feed stream and the second feed stream where the first feed stream has a temperature colder than the second feed stream, the packed bed cold absorber comprising at least a first and a second packed bed and producing an absorber overhead stream, an absorber bottoms stream, and an absorber side draw stream, the absorber side draw stream being removed between the first and the second packed beds;
(c) a first expander for expanding the absorber overhead stream;
(d) a fractionation tower for separating a first fractionation tower feed stream, the absorber overhead stream as a second fractionation tower feed stream, the absorber bottoms stream as a third fractionation tower feed stream, and a fractionation tower reflux stream, the fractionation tower producing a fractionation tower overhead stream that contains substantially all the methane and lighter components and a fractionation tower bottoms stream that contains at least 95% of the C2+ components;
(e) a first heater for warming the fractionation tower overhead stream;
(f) a first compressor for compressing the fractionation tower overhead stream to produce a residue gas stream;
(g) a second cooler for cooling the at least a portion of the residue gas stream; and
(h) a third cooler for cooling and at least partially condensing the absorber side draw stream to form the first fractionation tower feed stream.

11. The apparatus of claim 10, further including a fourth cooler for cooling and at least partially condensing at least a portion of the inlet gas stream.

12. The apparatus of claim 10 or claim 11, wherein the first cooler, the second cooler, the third cooler, and the first heater comprise a single heat exchanger that provides heat exchange contact with each of these streams.

13. The apparatus of any one of claims 10 to 12, further including a second expander for expanding at least a portion of the absorber bottoms stream prior to being sent to the fractionation tower.

14. The apparatus of any one of claims 10 to 13, further including a third expander for expanding at least a portion of the residue recycle stream prior to being sent to the fractionation tower.

15. The apparatus of any one of claims 10 to 14, further including a fourth expander for expanding at least a portion of the absorber side draw stream prior to being sent to the fractionation tower.

## Patentansprüche

1. Verfahren zum Trennen eines Einlassgasstroms, welcher Methan, C2-Komponenten, C3-Komponenten und schwerere Kohlenwasserstoffe enthält, in eine flüchtige Gasfraktion, die im Wesentlichen alles Methan enthält, und eine weniger flüchtige Kohlenwasserstofffraktion, die zumindest 95% der C2+-Komponenten enthält, wobei der Vorgang die folgenden Schritte umfasst:
(a) Teilen eines Einlassgasstroms in einen ersten Einspeisungsstrom und einen zweiten Einspeisungsstrom und Abkühlen des ersten und zweiten Einspeisungsstroms;
(b) Zuführen eines oberen Teils eines Füllkörperbett-Kälteabsorptionsmittels mit dem ersten Einspeisungsstrom und eines unteren Teils des Füllkörperbett-Kälteabsorptionsmittels mit dem zweiten Einspeisungsstrom, wobei der erste Einspeisungsstrom eine Temperatur hat, die kälter als der zweite Einspeisungsstrom ist, wobei das Füllkörperbett-Kälteabsorptionsmittel zumindest ein erstes und ein zweites Füllkörperbett umfasst und einen Absorptionsmittel-Überkopfstrom, einen Absorptionsmittel-Bodenstrom und einen Absorptionsmittel-Seitenabzugsstrom erzeugt;
(c) Entfernen des Absorptionsmittel-Seitenabzugsstroms aus dem Füllkörperbett-Kälteabsorptionsmittel;
(d) Kühlen und zumindest teilweise Verdichten des Absorptionsmittel-Seitenabzugsstroms, um einen ersten Fraktionierungsturm-Einspeisungsstrom zu bilden;
(e) Erweitern und anschließend Versorgen eines Fraktionierungsturms mit dem Absorptionsmittel-Überkopfstrom als zweiten Fraktionierungsturm-Einspeisungsstrom;
(f) Versorgen des Fraktionierungsturms mit dem Absorptions-Bodenstrom als dritten Fraktionierungsturm-Einspeisungsstrom;
(g) Trennen eines ersten Fraktionierungsturm-Einspeisungsstroms, des zweiten Fraktionierungsturm-Einspeisungsstroms, des dritten Fraktionierungsturms und des Fraktionierungsturm-Rückflussstroms zur Erzeugung eines Fraktionierungsturm-Überkopfstroms, der im Wesentlichen alles Methan und leichtere Komponenten enthält, und eines Fraktionierungsturm-Bodenstroms, der zumindest 95% der C2+-Komponenten enthält;
(h) Erwärmen und Komprimieren des Fraktionierungsturm-Überkopfstroms zur Erzeugung eines Rückstandsgasstroms;
(i) Entfernen zumindest eines Anteils des Rückstandsgasstroms als Fraktionierungsturm-Rückflussstrom;
(j) Abkühlen und Zuführen des Fraktionierungsturm-Rückflussstroms zum Fraktionierungsturm als Rückstands-Recyclingstrom; und
(k) Versorgen des Fraktionierungsturms mit dem ersten Fraktionierungsturm-Einspeisungsstrom, wodurch eine Menge des Rückstandsgases komprimiert und eine Menge des Rückstands-Recyclinggases an den Fraktionierungsturm gesendet wird.

2. Vorgang nach Anspruch 1, wobei der Schritt des Entfernens des Absorptionsmittel-Seitenabzugsstroms von dem Füllkörperbett-Kälteabsorptionsmittel das Entfernen des Absorptionsmittel-Seitenabzugsstroms zwischen dem ersten und dem zweiten Füllkörperbett einschließt.

3. Vorgang nach Anspruch 1 oder Anspruch 2, wobei der Schritt des Teilens des Einlassgasstroms das Teilen des Einlassgasstroms derart beinhaltet, dass der erste Einspeisungsstrom ungefähr 70% des Einlassgasstroms und der zweite Einspeisungsstrom ungefähr 30% des Einlassgasstroms enthält.

4. Vorgang nach irgendeinem der Ansprüche 1 bis 3, wobei der Schritt des Abkühlens des ersten und zweiten Einspeisungsstroms die folgenden Schritte umfasst:
(a) Abkühlen des ersten Einspeisungsstroms durch Wärmeaustauschkontakt mit einem Strom, der aus der Gruppe ausgewählt ist, die aus dem Absorptionsmittel-Seitenabzugsstrom, dem Rückstandsrecyclingstrom, dem Fraktionierungsturm-Überkopfstrom und Kombinationen von diesen besteht; und
(b) Abkühlen des zweiten Einspeisungsstroms durch Wärmeaustauschkontakt mit einem Strom, der aus der Gruppe ausgewählt ist, die aus einem ersten Verdampferbodenstrom, einem zweiten Verdampferbodenstrom und Kombinationen davon besteht.

5. Vorgang nach irgendeinem der Ansprüche 1 bis 4, wobei der Schritt des Abkühlens und zumindest teilweisen Verdichtens des Absorptionsmittel-Seitenabzugsstroms das Abkühlen des Absorptionsmittel-Seitenabzugsstroms derart einschließt, dass im Wesentlichen der gesamte Absorptionsmittel-Seitenabzugsstrom in der Flüssigphase ist.

6. Vorgang nach irgendeinem der Ansprüche 1 bis 5, ferner mit dem Schritt des Ausdehnens des Absorptionsmittelbodenstroms, bevor er dem Fraktionierungsturm zugeführt wird.

7. Vorgang nach irgendeinem der Ansprüche 1 bis 6, ferner mit dem Schritt des Erweiterns des Absorptionsmittel-Seitenabzugsstroms, bevor er dem Fraktionierungsturm zugeführt wird.

8. Vorgang nach irgendeinem der Ansprüche 1 bis 7, ferner mit dem Schritt des Erweiterns des Rückstandsrecyclingstroms, bevor er dem Fraktionierungsturm zugeführt wird.

9. Vorgang nach irgendeinem der Ansprüche 1 bis 8, ferner mit dem Schritt des Hinzufügens des Absorptionsmittel-Seitenabzugsstroms zu dem Rückstandsrecyclingstrom, um den ersten Fraktionierungsturm-Einspeisungsstrom zu bilden.

10. Vorrichtung zum Trennen eines Einlassgasstroms, welcher Methan, C2-Komponenten, C3-Komponenten und schwerere Kohlenwasserstoffe enthält, in eine flüchtige Gasfraktion, die im Wesentlichen alles Methan enthält, und eine weniger flüchtige Kohlenwasserstofffraktion, die zumindest 95% der C2+-Komponenten enthält, wobei die Vorrichtung umfasst:
(a) eine erste Kühlvorrichtung zum Abkühlen eines ersten Einspeisungsstroms und eines zweiten Einspeisungsstroms;
(b) ein Füllkörperbett-Kälteabsorptionsmittel zum Empfangen des ersten Einspeisungsstroms und des zweiten Einspeisungsstroms, wobei der erste Einspeisungsstrom eine Temperatur aufweist, die kühler als der zweite Einspeisungsstrom ist, wobei das Füllkörperbett-Kälteabsorptionsmittel zumindest ein erstes und ein zweites Füllkörperbett umfasst und einen Absorptionsmittel-Überkopfstrom, einen Absorptionsmittel-Bodenstrom und einen Absorptionsmittel-Seitenabzugsstrom erzeugt, wobei der Absorptionsmittel-Seitenabzugsstrom zwischen dem ersten und zweiten Füllkörperbett entfernt ist;
(c) ein erstes Erweiterungsmittel zum Erweitern des Absorptions-Überkopfstroms;
(d) einen Fraktionierungsturm zum Trennen eines ersten Fraktionierungsturm-Einspeisungsstroms, des Absorptionsmittel-Überkopfstroms als zweiten Fraktionierungsturm-Einspeisungsstrom, des Absorptionsmittel-Bodenstroms als dritten Fraktionierungsturm-Einspeisungsstrom und eines Fraktionierungsturm-Rückflussstroms, wobei der Fraktionierungsturm einen Fraktionierungsturm-Überkopfstrom erzeugt, der im Wesentlichen alles Methan und leichtere Komponenten enthält, und einen Fraktionierungsturm-Bodenstrom erzeugt, der zumindest 95% der C2+-Komponenten enthält;
(e) eine erste Heizvorrichtung zum Erwärmen des Fraktionierungsturm-Überkopfstroms;
(f) einen ersten Kompressor zum Komprimieren des Fraktionsturm-Überkopfstroms zur Erzeugung eines Rückstandsgasstroms;
(g) eine zweite Kühlvorrichtung zum Abkühlen des zumindest eines Anteils des Rückstandsgasstroms; und
(h) eine dritte Kühlvorrichtung zum Abkühlen und zumindest teilweisen Verdichten des Absorptionsmittel-Seitenabzugsstroms zum Bilden des ersten Fraktionierungsturm-Einspeisungsstroms.

11. Vorrichtung nach Anspruch 10, ferner mit einer vierten Kühlvorrichtung zum Abkühlen und zumindest teilweise Verdichten zumindest eines Anteils des Einlassgasstroms.

12. Vorrichtung nach Anspruch 10 oder Anspruch 11, wobei die erste Kühlvorrichtung, die zweite Kühlvorrichtung, die dritte Kühlvorrichtung und die erste Heizvorrichtung einen einzelnen Wärmetauscher umfassen, der einen Wärmetauschkontakt mit jedem dieser Ströme bereitstellt.

13. Vorrichtung nach irgendeinem der Ansprüche 10 bis 12, ferner mit einer zweiten Erweiterungsvorrichtung zum Erweitern zumindest eines Anteils des Absorptionsmittel-Bodenstroms, bevor er zum Fraktionierungsturm gesendet wird.

14. Vorrichtung nach irgendeinem der Ansprüche 10 bis 13, ferner mit einer dritten Erweiterungsvorrichtung zum Erweitern zumindest eines Anteils des Rückstandsrecyclingstroms, bevor er zum Fraktionierungsturm gesendet wird.

15. Vorrichtung nach irgendeinem der Ansprüche 10 bis 14, ferner mit einer vierten Erweiterungsvorrichtung zum Erweitern zumindest eines Anteils des Absorptionsmittel-Seitenabzugsstroms, bevor er zum Fraktionierungsturm gesendet wird.

## Revendications

1. Procédé de séparation d'un flux gazeux d'entrée contenant du méthane, des composants de C2, des composants de C3 et des hydrocarbures plus lourds en une fraction de gaz volatiles contenant essentiellement tout le méthane et en une fraction d'hydrocarbures moins volatiles contenant au moins 95 % des composants de C2+, le procédé comprenant les étapes consistant à :
(a) fractionner un flux gazeux d'entrée en un premier flux d'alimentation et en un deuxième flux d'alimentation, et refroidir les premier et deuxième flux d'alimentation ;
(b) fournir le premier flux d'alimentation à la partie haute d'un absorbeur de froid à lits fixes et le deuxième flux d'alimentation à la partie basse de l'absorbeur de froid à lits fixes, le premier flux d'alimentation ayant une température inférieure à celle du deuxième flux d'alimentation, l'absorbeur de froid à lits fixes comprenant au moins des premier et deuxième lits fixes et produisant un flux de distillat de tête d'absorbeur, un flux de produits de fond d'absorbeur et un flux d'extraction latérale d'absorbeur ;
(c) éliminer le flux d'extraction latérale d'absorbeur de l'absorbeur de froid à lits fixes ;
(d) refroidir et au moins partiellement condenser le flux d'extraction latérale d'absorbeur afin de former un premier flux d'alimentation de tour de fractionnement ;
(e) détendre puis fournir le flux de distillat tête d'absorbeur à une tour de fractionnement en guise de deuxième flux d'alimentation de tour de fractionnement ;
(f) fournir le flux de produits de fond d'absorbeur à la tour de fractionnement en guise de troisième flux d'alimentation de tour de fractionnement ;
(g) séparer le premier flux d'alimentation de tour de fractionnement, le deuxième flux d'alimentation de tour de fractionnement, le troisième flux d'alimentation de tour de fractionnement et le flux de reflux de tour de fractionnement afin de produire un flux de distillat de tête de tour de fractionnement contenant :
essentiellement la totalité du méthane, des composants plus légers et un flux de produits de fond de tour de fractionnement contenant au moins 95 % des composants de C2+ ;
(h) chauffer et comprimer le flux de distillat de tête de tour de fractionnement afin de produire un flux de gaz résiduaires ;
(i) éliminer au moins une partie du flux de gaz résiduaires en guise de flux de reflux de tour de fractionnement ;
(j) refroidir et fournir le flux de reflux de tour de fractionnement à la tour de fractionnement en guise de flux de recyclage de résidus ; et
(k) fournir le premier flux d'alimentation de tour de fractionnement à la tour de fractionnement, en réduisant ainsi une quantité des gaz résiduaires comprimés et une quantité des gaz de recyclage de résidus envoyés à la tour de fractionnement.

2. Procédé selon la revendication 1, dans lequel l'étape d'élimination du flux d'extraction latérale d'absorbeur de l'absorbeur de froid à lits fixes comprend l'élimination du flux d'extraction latérale d'absorbeur entre les premier et deuxième lits fixes.

3. Procédé selon la revendication 1 ou 2, dans lequel l'étape consistant à fractionner le flux gazeux d'entrée comprend le fractionnement du flux gazeux d'entrée de telle sorte que le premier flux d'alimentation comporte environ 70 % du flux gazeux d'entrée et que le deuxième flux d'alimentation comporte environ 30 % du flux gazeux d'entrée.

4. Procédé selon l'une quelconque des revendications 1 à 3, dans lequel l'étape de refroidissement des premier et deuxième flux d'alimentation comprend les étapes consistant à :
(a) refroidir le premier flux d'alimentation par échange de chaleur par contact avec un flux choisi dans le groupe constitué du flux d'extraction latérale d'absorbeur, du flux de recyclage de résidus, du flux de distillat de tête de tour de fractionnement et de leurs combinaisons ; et
(b) refroidir le deuxième flux d'alimentation par échange de chaleur par contact avec un flux choisi dans le groupe constitué d'un premier flux de produits de fond de rebouilleur, d'un deuxième flux de produits de fond de rebouilleur et de leurs combinaisons.

5. Procédé selon l'une quelconque des revendications 1 à 4, dans lequel l'étape de refroidissement et, au moins partiellement, de condensation du flux d'extraction latérale d'absorbeur comprend le refroidissement du flux d'extraction latérale d'absorbeur de telle sorte que pratiquement la totalité du flux d'extraction latérale d'absorbeur soit en phase liquide.

6. Procédé selon l'une quelconque des revendications 1 à 5, comprenant en outre l'étape consistant à détendre le flux de produits de fond d'absorbeur avant d'alimenter avec la tour de fractionnement.

7. Procédé selon l'une quelconque des revendications 1 à 6, comprenant en outre l'étape consistant à détendre le flux d'extraction latérale d'absorbeur avant d'alimenter avec la tour de fractionnement.

8. Procédé selon l'une quelconque des revendications 1 à 7, comprenant en outre l'étape consistant à détendre le flux de recyclage de résidus avant d'alimenter avec la tour de fractionnement.

9. Procédé selon l'une quelconque des revendications 1 à 8, comprenant en outre l'étape consistant à ajouter le flux d'extraction latérale d'absorbeur au flux de recyclage de résidus afin de former le premier flux d'alimentation de tour de fractionnement.

10. Appareil de séparation d'un flux gazeux d'entrée contenant du méthane, des composants de C2, des composants de C3 et des hydrocarbures plus lourds en une fraction de gaz volatiles contenant essentiellement tout le méthane et en une fraction d'hydrocarbures moins volatiles contenant au moins 95 % des composants de C2+, l'appareil comprenant :
(a) un premier refroidisseur pour refroidir un premier flux d'alimentation et un deuxième flux d'alimentation ;
(b) un absorbeur de froid à lits fixes pour recevoir le premier flux d'alimentation et le deuxième flux d'alimentation, le premier flux d'alimentation ayant une température inférieure à celle du deuxième flux d'alimentation, l'absorbeur de froid à lits fixes comprenant au moins des premier et deuxième lits fixes et produisant un flux de distillat de tête d'absorbeur, un flux de produits de fond d'absorbeur et un flux d'extraction latérale d'absorbeur, le flux d'extraction latérale d'absorbeur étant éliminé entre les premier et deuxième lits fixes ;
(c) un premier détendeur pour détendre le flux de distillat de tête d'absorbeur ;
(d) une tour de fractionnement pour séparer un premier flux d'alimentation de tour de fractionnement, le flux de distillat de tête d'absorbeur en guise de deuxième flux d'alimentation de tour de fractionnement, le flux de produits de fond d'absorbeur en guise de troisième flux d'alimentation de tour de fractionnement, et un flux de reflux de tour de fractionnement, la tour de fractionnement produisant un flux de distillat de tête de tour de fractionnement contenant essentiellement la totalité du méthane et des composants plus légers, et un flux de produits de fond de tour de fractionnement contenant au moins 95 % des composants de C2+ ;
(e) un premier réchauffeur pour chauffer le flux de distillat de tête de tour de fractionnement ;
(f) un premier compresseur pour comprimer le flux de distillat de tête de tour de fractionnement afin de produire un flux de gaz résiduaires ;
(g) un deuxième refroidisseur pour refroidir une partie au moins du flux de gaz résiduaires ; et
(h) un troisième refroidisseur pour refroidir et au moins partiellement condenser le flux d'extraction latérale d'absorbeur afin de former le premier flux d'alimentation de tour de fractionnement.

11. Appareil selon la revendication 10, comprenant en outre un quatrième refroidisseur pour refroidir et au moins partiellement condenser au moins une partie du flux gazeux d'entrée.

12. Appareil selon la revendication 10 ou 11, dans lequel le premier refroidisseur, le deuxième refroidisseur, le troisième refroidisseur et le premier réchauffeur comprennent un échangeur de chaleur unique permet un échange de chaleur par contact avec chacun de ces flux.

13. Appareil selon l'une quelconque des revendications 10 à 12, comprenant en outre un deuxième détendeur pour détendre au moins une partie du flux de produits de fond d'absorbeur avant de commencer à l'envoyer à la tour de fractionnement.

14. Appareil selon l'une quelconque des revendications 10 à 13, comprenant en outre un troisième détendeur pour détendre au moins une partie du flux de recyclage de résidus avant de commencer à l'envoyer à la tour de fractionnement.

15. Appareil selon l'une quelconque des revendications 10 à 14, comprenant en outre un quatrième détendeur pour détendre au moins une partie du flux d'extraction latérale d'absorbeur avant de commencer à l'envoyer à la tour de fractionnement.
